# EUROPEAN PATENT SPECIFICATION

(11) **EP 4 470 511 B1**
(45) Date of publication and mention of the grant of the patent: **23.09.2026**
(21) Application number: 23746240.3
(22) Date of filing: 19.01.2023
(51) Int. Cl.: A61F 2/954, A61F 2/07

(54) **BRANCH SHEATH AND INTRAVASCULAR STENT DELIVERY DEVICE**
ABZWEIGHÜLLE UND VORRICHTUNG ZUR EINFÜHRUNG EINES INTRAVASKULÄREN STENTS
GAINE DE RAMIFICATION ET DISPOSITIF DE POSE D'ENDOPROTHÈSE INTRAVASCULAIRE

(30) Priority: 26.01.2022 CN 202210089604
(43) Date of publication of application: 04.12.2024
(73) Proprietor: Shanghai MicroPort Endovascular MedTech (Group) Co., Ltd., Shanghai 201318 (CN)
(72) Inventor: WU, Weiyi, Shanghai 201318 (CN); ZHAO, Mingjie, Shanghai 201318 (CN); ZHU, Yongfeng, Shanghai 201318 (CN); SHAN, Wenwen, Shanghai 201318 (CN); ZHANG, Bowei, Shanghai 201318 (CN); ZHU, Qing, Shanghai 201318 (CN); ZHAO, Shuhua, Shanghai 201318 (CN)
(74) Representative: Patentship Patentanwaltsgesellschaft mbH
(86) International application number: PCT/CN2023/073121
(87) International publication number: WO 2023/143359

(56) References cited:
- CN-A- 102 415 924
- CN-A- 110 314 014
- CN-A- 110 811 945
- CN-A- 113 633 447
- CN-A- 114 099 100
- CN-B- 113 017 952
- CN-U- 208 851 712
- US-A1- 2021 161 694

## Description

### TECHNICAL FIELD

The present invention relates to the technical field of medical instruments and, in particular, to a branch sheath and an intravascular stent delivery device.

### BACKGROUND

With the development of intraluminal devices and minimally invasive interventional therapy, intravascular stent placement has become a common treatment method for aortic arch diseases, which aims to utilize a special delivery system to deliver a covered stent to a diseased site so that the covered stent can be released at the diseased site to isolate the aneurysm, tear or dissection from the blood, avoiding the risk of death due to bleeding derived from aneurysm rupture and dissection expansion.

Although the minimally invasive interventional therapy using a covered stent have the advantage of less trauma than thoracotomy, there are still some problems, especially in the handling of branch vessels. At present, the Castor stent adopts the integrated design of the branch stent and the main stent. Before the implantation of the main stent, the guide catheter and the guide wire are introduced through the puncture of the branch vessel of the upper limb. The guide wire for the branch sheath can therefore enter the branch vessel smoothly through the guide passageway. In this case, there is no need to select the branch vessel after the main stent enters the blood vessel. However, this integrated design may cause the branch stent to be moved by the main stent, resulting in the problem of displacement of the branch stent. During the release process of the main stent, the corresponding guide passageway for the branch stent is established, and the branch stent is implanted after the conveyor for the main stent is withdrawn. During this process, the main stent has lost the support from the conveyor, requiring an additional anchor point to prevent it from displacement during the release process of the branch stent. Moreover, the integrated branch stent also needs to be pulled to be prevented from being displaced into the aortic lumen.

In addition, during the process of introducing and releasing the stent, the stent delivery device will inevitably come into contact with the released main stent, especially at the aortic arch with a large curvature, and the tapered head of the delivery device will rub against the main stent. The friction of the stent itself cannot ensure that the stent can be fixed on the vessel wall. If the main stent is moved, the positioning of the branch stent may be inaccurate. The movement of the stent will also damage the vessel wall, and even cause the branch stent that has entered the branch vessel to slip out of the innominate branch vessel and enter the aortic lumen, causing the innominate branch vessel to be blocked. This situation would be very dangerous and would require emergency open surgery.

Therefore, a new branch sheath and a new intravascular stent delivery device are needed to avoid the above-mentioned problems.

CN 113 017 952 B relates to a branch sheath and delivery system.

CN 208 851 712 U relates to a intravascular stent and its traction device.

### SUMMARY OF THE INVENTION

An objective of the present invention is to provide a branch sheath and an intravascular stent delivery device to solve the problem of how to prevent the branch stent from displacement during the delivery process of the stent.

In order to solve the above technical problems, the present invention provides a branch sheath, comprising a connecting tube, a sleeving, a control wire, a restraining coil and a first fixing coil; wherein
a distal end of the connecting tube is connected to a proximal end of the sleeving; the control wire extends through the connecting tube and the sleeving, and the control wire has a proximal end and a distal end respectively extending out of the connecting tube and the sleeving;
a proximal end of the restraining coil is connected to the distal end of the connecting tube, and a distal end of the restraining coil passes through and extends out of the sleeving so as to pass through and extend out of an aperture of a branch stent to pull and secure a bare end of the branch stent; the distal end of the control wire extends through a part of the restraining coil extending out of the bare end from the aperture therein;
a proximal end of the first fixing coil is connected to the distal end of the connecting tube, and a distal end of the first fixing coil is configured to connect to a main stent.

The control wire is configured to be pulled away from the straining coil, the sleeving and the connecting tube to release the branch stent. Optionally, in the branch sheath, the proximal end of the control wire is fixedly connected to a stopper so as to prevent the proximal end of the control wire from sliding.

Optionally, in the branch sheath, the stopper is detachably connected to the proximal end of the connecting tube.

Optionally, in the branch sheath, the detachable connection comprises sleeve connection using a heat-shrinkable tube or bio-glue adhesion.

Optionally, in the branch sheath, the stopper is integrally formed with the connecting tube, and a crease is formed at a connection between the stopper and the connecting tube, so that the connection can be made to be broken along the crease during release of the branch stent and the control wire can be pulled out.

Optionally, in the branch sheath, the stopper is a heat-shrinkable tube sleeved on the proximal end of the connecting tube, and the proximal end of the control wire is wrapped in the stopper.

Optionally, in the branch sheath, the branch sheath further comprises a second fixing coil; wherein a proximal end of the second fixing coil is connected to the distal end of the connecting tube, and a distal end of the second fixing coil extends through the sleeving, and wherein the distal end of the restraining coil extends out of the aperture of the bare end, and further extends through and is hooked on the distal end of the second fixing coil to form a first extension through which the control wire extends.

Optionally, in the branch sheath, a number of the first fixing coils is greater than or equal to 2, and the first fixing coils are arranged at an interval along a circumference of the connecting tube.

Optionally, in the branch sheath, at least one of the first fixing coils has a knot; wherein the distal end of the restraining coil extends out of the aperture of the bare end, further extends through a hole formed by the knot at a side closer to the connecting tube, and is hooked on the knot to form a second extension through which the control wire extends.

Optionally, in the branch sheath, the control wire has an axial length that is greater than or equal to 10 mm from the intersection between the part of the restraining coil extending out of the aperture of the bare end and the control wire to the distal end of the control wire.

Optionally, in the branch sheath, the restraining coil has a length greater than a length of the first fixing coil, a length of the part of the restraining coil extending out of the sleeving is greater than or equal to 15 mm, and a part of the first fixing coil extends out of the sleeving and has a length greater than or equal to 10 mm.

Optionally, the sleeving is a covered sleeving.

Based on the same inventive concept, the present invention also provides an intravascular stent delivery device, comprising a guide wire for main stent and at least one branch sheath as mentioned above; wherein the guide wire extends along an axial direction of the main stent through the first fixing coil connected to the main stent.

Optionally, in the intravascular stent delivery device, a length of the first fixing coil is greater than or equal to an axial length of the compressed branch stent plus a diameter of the compressed main stent.

In conclusion, the present invention provides a branch sheath and an intravascular stent delivery device. The branch sheath comprises a connecting tube, a sleeving, a control wire, a restraining coil and a first fixing coil. A distal end of the connecting tube is connected to a proximal end of the sleeving; the control wire extends through the connecting tube and the sleeving, and the control wire has a proximal end and a distal end respectively extending out of the connecting tube and the sleeving; a proximal end of the restraining coil is connected to the distal end of the connecting tube, and a distal end of the restraining coil passes through and extends out of the sleeving so as to extend out of a branch stent from an aperture therein to pull and secure a bare end of the branch stent. This prevents the branch stent from being pulled by the main stent to cause a displacement after the main stent is released. The control wire extends through the part of the restraining coil that extends out of the bare end from the aperture therein, so that the control wire can be pulled out of the restraining coil, the sleeving and the connecting tube after the position for release is determined, achieving a accurate release of the branch stent. After the branch stent is out of the predetermined position during the operation, it can also be pulled back into the branch vessel by the control wire at any time, achieving a precise positioning. The proximal end of the first fixing coil is connected with the distal end of the connecting tube, and the distal end of the first fixing coil is connected with the main stent, which enables the branch sheath to be delivered as a whole.

Therefore, the branch sheath and intravascular stent delivery device according to the present invention can not only prevent the branch stent from displacement and ensure an accurate position for the release, but also be operated easily with an increased implementability.

### BRIEF DESCRIPTION OF THE DRAWINGS

Fig. 1 is a structural schematic diagram of a branch sheath according to an embodiment of the present invention;
Fig. 2 is a schematic diagram showing a connection between a branch sheath and a main stent according to an embodiment of the present invention;
Fig. 3 is a schematic diagram of the bare end restrained by the restraining coil according to an embodiment of the present invention;
Fig. 4 is a schematic diagram showing a connection between a branch sheath and a main stent according to an embodiment of the present invention;
Fig. 5 is a structural schematic diagram of the restraining coil hooked on the second fixing coil according to an embodiment of the present invention;
Fig. 6 is a schematic diagram showing a connection between a branch sheath and a main stent according to an embodiment of the present invention;
Fig. 7 is a structural schematic view of the restraining coil hooked on the knot according to an embodiment of the present invention;
Figs. 8-10 are schematic diagrams showing the release of the branch stent according to an embodiment of the present invention.

List of reference signs:
11: control wire; 111: intersection;
21: connecting tube; 211: fixing tube;
22: covered sleeving;
23: first fixing coil; 231: knot;
24: restraining coil; 241: first extension; 242: second extension;
25: second fixing coil;
31: main stent;
32: guide wire for main stent;
41: branch stent; 411: bare end.

### DETAILED DESCRIPTION

In order to make the objectives, advantages and features of the present invention clearer, the present invention will be further described in detail below in conjunction with the accompanying drawings and specific embodiments. It should be noted that the drawings are all in very simplified form and not drawn to scale, and are only used to facilitate illustrating the embodiments of the present invention clearly. In addition, the structures shown in the drawings are often a part of the actual structures. In particular, each drawing focuses to display different parts, and sometimes uses different scales. It should also be understood that, unless otherwise specified or pointed out, the terms "first", "second", "third" and other descriptions in the specification are only used to distinguish each component, element, step, etc. in the specification, rather than being used to express the logical relationship or sequence relationship between various components, elements, and steps.

The definitions of "proximal end" and "distal end" here are that the "distal end" generally refers to the end of the medical device that first enters the patient's body during normal operation, and the "proximal end" generally refers to the end of the medical device that is closer to the operator during the normal operation.

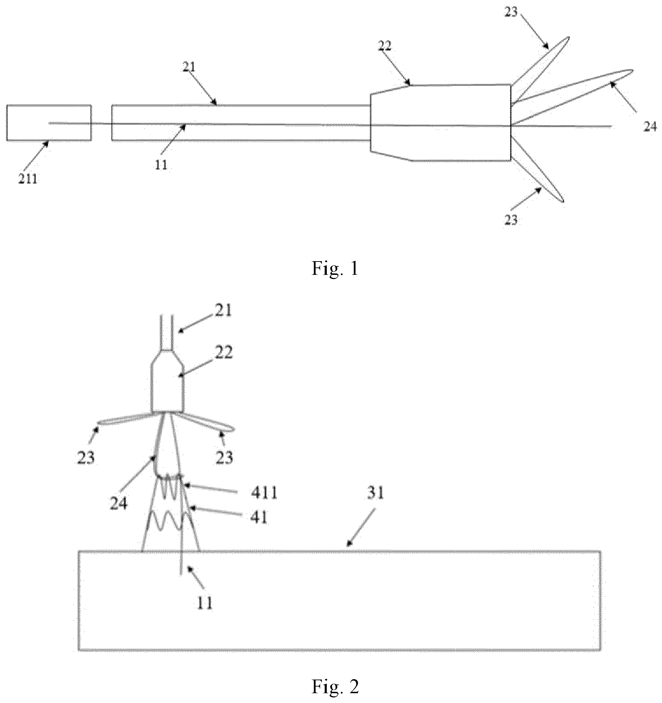

In order to solve the above technical problems, this embodiment provides a branch sheath. Please refer to Figs. 1-2, the branch sheath includes a control wire 11, a connecting tube 21, a sleeving, a first fixing coil 23 and a restraining coil 24. Optionally, the sleeving is used for an intravascular stent. Optionally, the sleeving is a covered sleeving 22. The following description is made by implementing the sleeving as the covered sleeving 22. A skilled person in the art shall know that any decription made to the covered sleeving 22 is also applicable for the sleeving. The distal end of the connecting tube 21 is connected to the proximal end of the covered sleeving 22. The control wire 11 extends through the connecting tube 21 and the covered sleeving 22. The two ends of the connecting tube 21 extends out of the connecting tube 21 and the covered sleeving 22 respectively. The proximal end of the restraining coil 24 is connected with the distal end of the connecting tube 21. The distal end of the restraining coil 24 passes through and extends out of the covered sleeving 22 so that the distal end of the restraining coil 24 is configured to extend out of the bare end 411 of the branch stent 41 from the aperture therein to pull and secure the bare end 411 of the branch stent 41. The control wire 11 extends through the part of the restraining coil 24 that extends out from the aperture of the bare end 411. The proximal end of the first fixing coil 23 is connected to the distal end of the connecting tube 21, and the distal end of the first fixing coil 23 is configured to connect with the main stent 31.

It can be seen that the branch sheath according to this embodiment secures the bare end 411 of the branch stent 41 through the pulling of the restraining coil 24, so as to prevent the branch stent 41 from displacement due to the dragging of the main stent when the branch stent 41 is released. Moreover, the control wire 11 extends through the part of the restraining coil 24 that extends out of the bare end 411 from the aperture therein, so that the control wire 11 can be pulled out of the restraining coil 24, the covered sleeving 22 and the connecting tube 21 during the release of the branch stent 41, achieving a accurate release of the branch stent 41. In addition, after the branch stent 41 is out of the predetermined position during the operation, it can also be pulled back into the branch vessel by the control wire 11 at any time, achieving a precise positioning.

The branch sheath will be described in detail below with reference to the accompanying Figs. 1-10.

Please refer to Figs. 1-3, the control wire 11 is configured to control the release of the branch stent 41. When the branch stent 41 is released, the control wire 11 is pulled out of the restraining coil 24, the covered sleeving 22 and the connecting tube 21, so as to complete the release of the branch stent 41. Before the branch stent 41 is released, the distal end of the control wire 11 extends through the connecting tube 21 and the covered sleeving 22 successively, and further extends through the part of the restraining coil 24 that extends out of the bare end 411 from the aperture therein. This narrows the area where the bare end 411 is movable, and ensures the stability in the pulling of the restraining coil 24. It also reduces the influence from the pulling of the main stent 31 on the branch stent 41, and avoids an early release of the branch stent 41. The branch stent 41 can be pulled back into the branch vessel at any time after leaving the predetermined position during the operation, achieving a precise positioning. The proximal end of the control wire 11 is fixedly connected with a stopper. Therefore, the proximal end of the control wire 11 is prevented from sliding into the connecting tube 21, or from being pulled out early by misoperation.

The stopper can be understood as a handle for pulling away the control wire 11, which on the one hand is convenient for the operator to perform a pulling action, and avoid misoperation which may cause an early release of the branch stent 41, and on another hand prevents the proximal end of the control wire 11 from being out of control and sliding into the connecting tube 21. In order to facilitate intraoperative operation, the connection between the stopper and the connecting tube 21 is a detachable connection. Optionally, the following three configurations can be applied to the stopper. One of the configurations is as shown in Fig. 1, the stopper is implemented as a fixing tube 211. The connection mode between the fixing tube 211 and the connecting tube 21 include but is not limited to the mode where one is sleeved on another by a heat-shrinkable tube or the mode where one is adhered to another by bio-glue. Specifically, the part of the control wire 11 extending out of the connecting tube 21 from the proximal end thereof is inserted into the fixing tube 211 and fixed in the fixing tube 211. When the control wire 11 needs to be pulled out, the connection between the fixing tube 211 and the proximal end of the connecting tube 21 is made to be broken, so that the control wire 11 can be pulled out and the branch stent 41 can be therefore released. Further, in order to facilitate the connection, the fixing tube 211 has an outer diameter same as that of the connecting tube 21. Optionally, the material of the fixing tube 211 includes nickel-titanium alloy.

A second one of the configurations is that a short section close to the proximal end of the connecting tube 21 is implemented as the stopper. In this case, the stopper is integrally formed with the connecting tube 21, and a smooth crease is formed by laser at the connection between the stopper and the connecting tube 21, so that the connection can be made to be broken along the smooth crease during the release of the branch stent 41, and thus the control wire 11 can be pulled out.

A third one of the configurations for the stopper is that the stopper is implemented as a heat-shrinkable tube. In this case, a short section of the control wire 11 extending out of the connecting tube 21 from the proximal end thereof is bent to form a zigzag shape. The heat-shrinkable tube is then used to wrap and sleeve the proximal end of the connecting tube 21, so as to prevent the proximal end of the control wire 11 from sliding into the connecting tube 21 or avoid the misoperation which may cause an early release of the branch stent 41. When the control wire 11 needs to be pulled out, the heat-shrinkable tube is cut open with a blade, and the control wire 11 can be directly pulled out.

Please continue to refer to Fig. 1, the connecting tube 21 serves as the basic skeleton of the branch sheath, and is configured to support and secure the covered sleeving 22, the first fixing coil 23 and the restraining coil 24. Further, both the connecting tube 21 and the covered covered sleeving 22 are hollow, that is, they each have an inner cavity extending from one end to the other. The distal end of the connecting tube 21 is connected to the proximal end of the covered sleeving 22, and the inner cavities of the connecting tube 21 and the covered sleeving 22 are in communication with each other. The covered sleeving 22 is configured to accommodate the compressed branch stent 41. Optionally, the materials of the control wire 11, the connecting tube 21 and the covered sleeving 22 include but are not limited to nickel-titanium alloy.

Please refer to Fig. 2, the proximal end of the restraining coil 24 is connected to the distal end of the connecting tube 21. The distal end of the restraining coil 24 extends through the covered sleeving 22, and further extends out of the bare end 411 of the branch stent 41 form the aperture therein, so as to bind the bare end 411 of the branch stent, and secure the compressed branch stent inside the covered sleeving 22. The stent according to this embodiment includes a main stent 31 and at least one branch stent 41. The end of the branch stent 41 away from the main stent 31 is a free end, which is called the bare end 411 according to this embodiment. Further, the branch stent 41 may also be a covered stent. Since the bare end 411 is used for connection, the bare end 411 is not covered with a membrane.

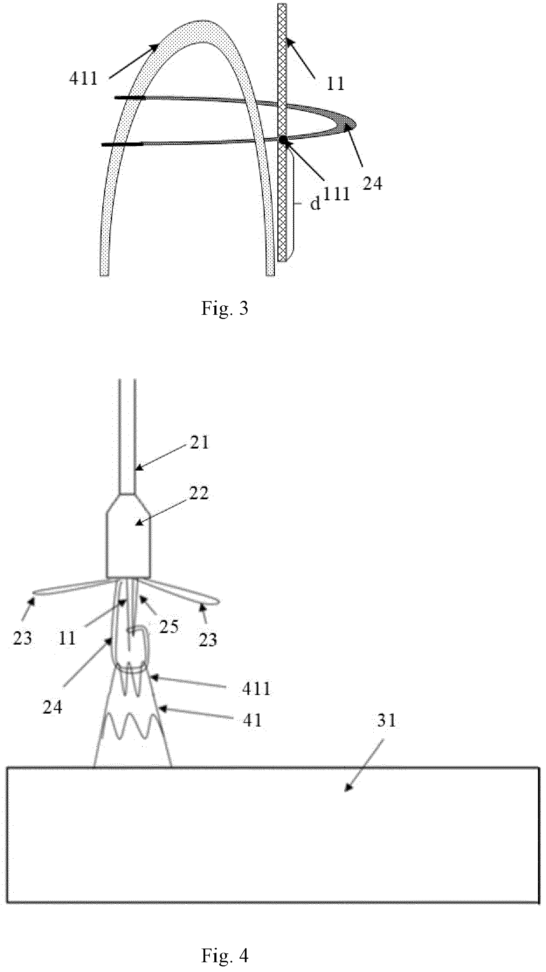

As shown in Fig. 3, the distal end of the restraining coil 24 extends through each aperture of the bare end 411 of the branch stent 41 sequentially, so as to pull and tighten the bare end 411 of the branch stent. This prevents the branch stent 41 from being pulled by the main stent 31 after the main stent 31 is released and thus avoids a displacement of the branch stent 41. As a result, an accurate position for the release can be ensured. The distal end of the control wire 11 is also inserted into the restraining coil 24 to further compress the bare end 411 of the branch stent, so that the branch stent 41 can be released easily by pulling the control wire 11 away under a small external force.

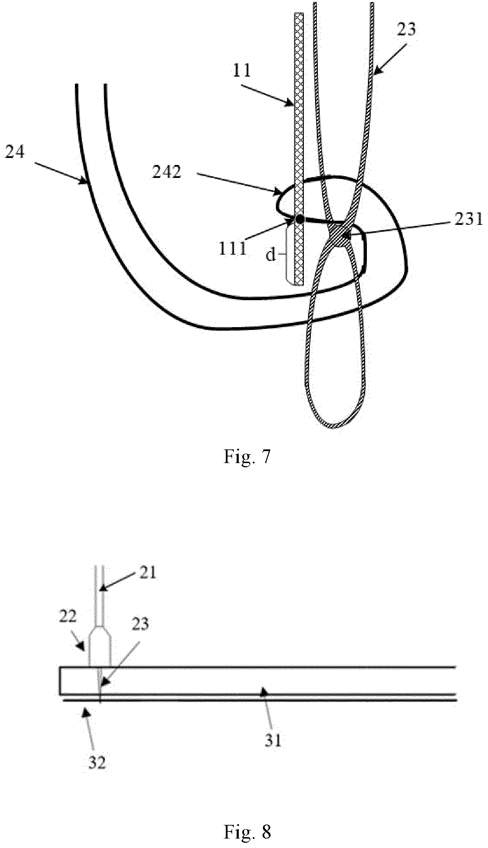

Please continue to refer to Fig. 2, the first fixing coil 23 is arranged in the branch sheath. The first fixing coil 23 is configured to fix the branch stent 41 and the main stent 31. Before the branch stent 41 is released, the proximal end of the first fixing coil 23 is connected to the distal end of the connecting tube 21, and the distal end of the first fixing coil 23 extends through the covered sleeving 22 to the main stent 31 to surround the main stent 31. As shown in Fig. 8, the guide wire 32 for the main stent extends through the distal end of the first fixing coil 23, so that the branch stent 41 and the main stent 31 are integrated for delivery as a whole. Therefore, the length of the first fixing coil 23 is generally greater than or equal to the axial length of the compressed branch stent 41 plus the diameter of the compressed main stent 31. Optionally, the part of the first fixing coil 23 extending out of the covered sleeving 22 has a length greater than or equal to 10 mm. Further, in order to achieve a stable connection between the branch stent 41 and the main stent 31, the number of the first fixing coil 23 may be greater than or equal to one, for example, 1, 2, 3 or 4. The first fixing coils 23 are arranged at an interval along the circumference of the connecting tube 21.

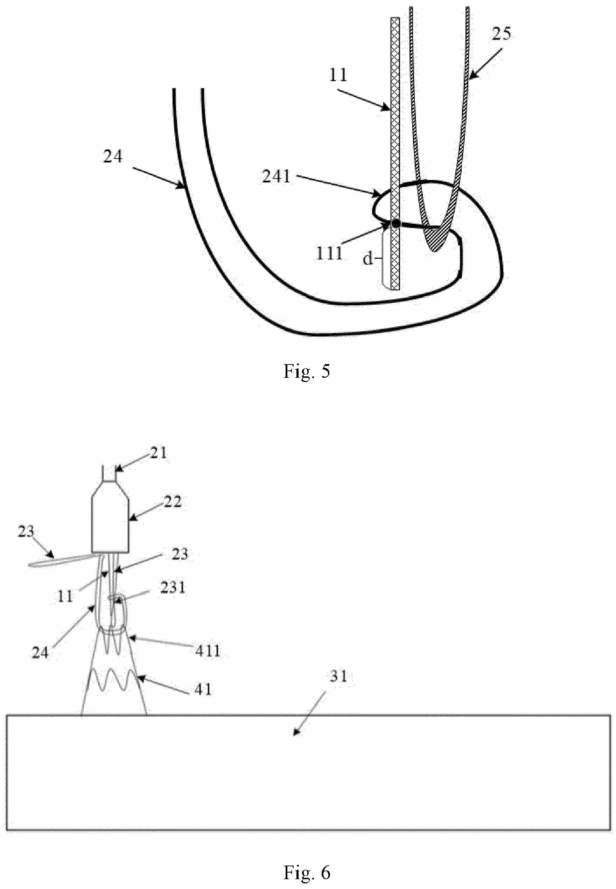

Please refer to Figs. 4 and 5, the control wire 11 will pass through the covered sleeving 22 when it is pulled away. Therefore, it is likely that the control wire 11 may puncture the covered sleeving 22 during the pulling process if the control wire 11 is too long. Therefore, in order to shorten the length of the control wire 11 and ensure that the covered sleeving 22 will not be damaged, the branch sheath according to this embodiment further includes a second fixing coil 25. The proximal end of the second fixing coil 25 extends through the covered sleeving 22 to connect with the distal end of the connecting tube 21. The distal end of the restraining coil 24 extends out of the bare end 411 from the aperture therein, and further extends through and is hooked on the distal end of the second fixing coil 25 to form a first extension 241 through which the control wire 11 extends. As shown in Fig. 4, the first extension 241 is in the shape of a hook, which is convenient for hooking on the second fixing coil 25. Therefore, the restraining coil 24 bends upwards and is hooked on the second fixing coil 25, and the restraining coil 24 extends upwards, so that the control wire 11 can be inserted in the restraining coil 24 at the position where the second fixing coil 25 locates. As a result, the control wire 11 is shortened, which avoids damage to the covered sleeving 22 caused by the control wire 11 being too long.

Based on the same concept, in order to reduce the cost and structural complexity, the second fixing coil 25 in Figs. 4 and 5 may not be provided. In this case, the first fixing coil 23 may be directly used to achieve the purpose of shortening the control wire 11. As shown in Figs. 6 and 7, the branch sheath is provided with two first fixing coils 23. One of the first fixing coils 23 is formed with a knot 231 thereon, thereby dividing the first fixing coil 23 into two connected coils each forming a hole. Certainly, according to the specification of the first fixing coils 23, it is also possible that a plurality of the first fixing coils 23 may be formed with knots. Preferably, the knot 231 is a dead knot. After the distal end of the restraining coil 24 extends out of the bare end 411 from the aperture therein, it further extends through the hole formed by the knot 231 at a side closer to the connecting tube 21, and is hooked on the knot 231 to form a second extension 242 through which the control wire 11 extends. In other words, the first fixing coil 23 formed with a knot has an upper hole and a lower hole. The upper hole is closer to the proximal end of the covered sleeving 22 and the proximal end of the connecting tube 21. The lower hole is closer to the main stent 31, and the coil corresponding to the lower hole still surrounds the main stent 31 for securing. The distal end of the restraining coil 24 extends out of the bare end 411, then through the upper hole, and is hooked on the knot 231 to form a second extension 242 through which the control wire 11 extends. In this way, the length of the control wire 11 is shortened, which avoids damage to the covered sleeving 22 caused by the control wire 11 being too long. In the case where a plurality of the first fixing coils 23 have knots 231, the second extension 242 extends through the upper holes in sequence to be hooked on a plurality of the knots 231, so as to improve the stability of the hooking of the restraining coil 24. Further, the second extension 242 is in the shape of a hook, which is convenient for hooking on the knot 231. The material used for the restraining coil 24 and the first fixing coil 23 includes polytetrafluoroethylene.

Further, in order to ensure that the branch sheath achieves a better technical effect, this embodiment limits the quantitative relationship among the control wire 11, the first fixing coil 23 and the restraining coil 24. As shown in Figs. 3, 5 and 7, the control wire 11 has an axial length d that is greater than or equal to 10 mm (e.g., 10 mm, 12 mm, or 14 mm) from the intersection 111 between the part of the restraining coil 24 extending out of the bare end 411 from the aperture therein and the control wire 11 to the distal end of the control wire. In other words, as shown in Fig. 3, after the restraining coil 24 extends out of the bare end 411 from the aperture therein, the control wire 11 extending through the restraining coil 24 intersects with the restraining coil 24 at the intersection 111, and then the control wire 11 further extends toward the branch stent 41. As a result, an axial distance d between the position where the distal end of the control wire 11 finally is and the intersection 111 is greater than or equal to 10 mm. The control wire 11 extends along the axis of the connecting tube 21, and the axial distance d is equal to the distance from the intersection 111 to the distal end of the control wire 11. As shown in Figs. 5 and 7, the distance d from the intersection 111 between the first or section extension 241, 242 and the control wire 11 to the position where the distal end of the control wire 11 finally is is greater than or equal to 10 mm. Thus, it can be ensured that the control wire 11 is prevented from being separated from the restraining coil 24 at a timing earlier than desired during the process of pulling the covered sleeving 22 and the connecting tube 21 to separate the branch stent 41 from the covered sleeving 22 before the branch stent is released. Further, in order to ensure that the restraining coil 24 pulls the bare end 411, and is stably hooked on the second fixing coil 25 (as shown in Fig. 5), or is hooked on the knot 231 (as shown in Fig. 7), the length of the restraining coil 24 is greater than the length of the first fixing coil 23. Preferably, the length of the part of the restraining coil 24 extending out of the covered sleeving 22 is greater than or equal to 15 mm (e.g., 15 mm, 18 mm, or 20 mm).

Based on the same inventive concept, this embodiment also provides an intravascular stent delivery device, please refer to Figs. 8-10, the intravascular stent delivery device includes a guide wire 32 for main stent and at least one branch sheath as mentioned above. As shown in Fig. 8, the guide wire 32 extends along the axial direction of the main stent 31 through the first fixing coil 23 connected to the main stent 31. In addition, the main stent 31 is further provided with a restraining wire configured to bind the compressed main stent 31, and the guide wire 32 also extends through the restrain wire.

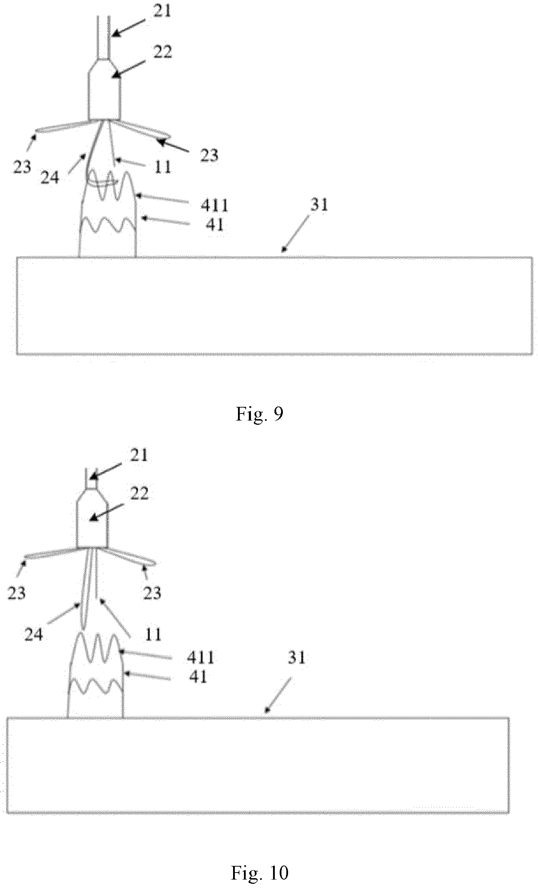

During the delivery of the intravascular stent, as shown in Fig. 8, the branch stent 41 is loaded in the branch sheath, and then the compressed main stent 31 is bundled, and then passed through the first fixing coil 23, so that the main stent 31 is connected to the branch sheath by the first fixing coil 23 and the guide wire 32. Then, the guide wire 32 extends along the axial direction of the compressed main stent 31 through the first fixing coil 23. The intravascular stent delivery device on which the main stent 31 and the branch stent 41 are loaded is delivered to the target position. After the position of the branch stent 41 is determined, the guide wire 32 is pulled out to release the main stent 31. Then, the covered sleeving 22 and the connecting tube 21 are pulled to separate the compressed branch stent 41 from the covered sleeving 22 so that the branch stent returns to its normal configuration. At this time, the control wire 11 will follow the covered sleeving 22 and the connecting tube 21 to move a certain distance, but is still kept extending through the restraining coil 24, and the restraining coil 24 still pulls and secures the bare end 411. Finally, as shown in Figs. 9 and 10, the control wire 11 is pulled out, so that the control wire 11 is separated from the restraining coil 24, and a force is applied to drive the restraining coil 24 to move, so that the bare end 411 of the branch stent 41 is free from the pulling of the restraining coil 24 to achieve the release of the branch stent 41.

In summary, in the branch sheath and intravascular stent delivery device according to the present embodiment, the proximal end of the restraining coil 24 in the branch sheath is connected to the distal end of the connecting tube 21, and the distal end of the restraining coil 24 passes through and extends out of the covered sleeving 22. The distal end of the restraining coil 24 is configured to extends out of the bare end 411 of the branch stent from the aperture therein, so as to pull and secure the bare end 411 of the compressed branch stent. This prevents the branch stent from being pulled by the main stent to cause a displacement after the main stent is released. The control wire 11 extends through the part of the restraining coil 24 that extends out of the bare end 411 from the aperture therein, so that the control wire 11 can be pulled out of the restraining coil 24, the covered sleeving 22 and the connecting tube 21 after the position for release is determined, achieving a accurate release of the branch stent 41. After the branch stent 41 is out of the predetermined position during the operation, it can also be pulled back into the branch vessel by the control wire 11 at any time, achieving a precise positioning. The proximal end of the first fixing coil 23 is connected with the distal end of the connecting tube 21, and the distal end of the first fixing coil 23 is connected with the main stent 31, which enables the branch sheath to be delivered as a whole. Therefore, the branch sheath and intravascular stent delivery device according to this embodiment can not only prevent the branch stent 41 from displacement and ensure an accurate position for the release, but also be operated easily with an increased implementability.

## Claims

1. A branch sheath, comprising a connecting tube (21), a sleeving, a control wire (11), a restraining coil (24) and a first fixing coil (23); wherein
a distal end of the connecting tube (21) is connected to a proximal end of the sleeving; the control wire (11) extends through the connecting tube (21) and the sleeving, and the control wire (11) extends along an axis of the connecting tube (21) and the sleeving (22), and has a proximal end and a distal end respectively extending out of the connecting tube (21) and the sleeving;
a proximal end of the restraining coil (24) is connected to the distal end of the connecting tube (21), and a distal end of the restraining coil (24) passes through and extends out of the sleeving so as to pass through and extend out of an aperture of a bare end (411) of the branch stent (41) to pull and secure the bare end (411) of the branch stent (41); the distal end of the control wire (11) extends through a part of the restraining coil (24) extending out of the aperture of the bare end (411), and wherein the control wire (11) is configured to be pulled out of the restraining coil (24), the covered sleeving (22) and the connecting tube (21) to release of the branch stent (41);
a proximal end of the first fixing coil (23) is connected to the distal end of the connecting tube (21), and a distal end of the first fixing coil (23) is configured to connect to a main stent (31).

2. The branch sheath according to claim 1, wherein the proximal end of the control wire (11) is fixedly connected to a stopper so as to prevent the proximal end of the control wire (11) from sliding.

3. The branch sheath according to claim 2, wherein the stopper is detachably connected to the proximal end of the connecting tube (21).

4. The branch sheath according to claim 3, wherein the detachable connection comprises sleeve connection using a heat-shrinkable tube or bio-glue adhesion.

5. The branch sheath according to claim 3, wherein the stopper is integrally formed with the connecting tube (21), and a crease is formed at a connection between the stopper and the connecting tube (21), so that the connection can be made to be broken along the crease during release of the branch stent (41) and the control wire (11) can be pulled out.

6. The branch sheath according to claim 3, wherein the stopper is a heat-shrinkable tube sleeved on the proximal end of the connecting tube (21), and the proximal end of the control wire (11) is wrapped in the stopper.

7. The branch sheath according to claim 1, further comprising a second fixing coil (25); wherein a proximal end of the second fixing coil (25) is connected to the distal end of the connecting tube (21), and a distal end of the second fixing coil (25) extends through the sleeving, and wherein the distal end of the restraining coil (24) extends out of the aperture of the bare end (411), and further extends through and is hooked on the distal end of the second fixing coil (25) to form a first extension (241) through which the control wire (11) extends.

8. The branch sheath according to any one of claims 1-7, wherein a number of the first fixing coils (23) is greater than or equal to 2, and the first fixing coils (23) are arranged at an interval along a circumference of the connecting tube (21).

9. The branch sheath according to claim 8, wherein at least one of the first fixing coils (23) has a knot (231); wherein the distal end of the restraining coil (24) extends out of the aperture of the bare end (411), further extends through a hole formed by the knot (231) at a side closer to the connecting tube (21), and is hooked on the knot (231) to form a second extension (242) through which the control wire (11) extends.

10. The branch sheath according to claim 1, wherein the control wire (11) has an axial length that is greater than or equal to 10 mm from an intersection (111) between the part of the restraining coil (24) extending out of the aperture of the bare end (411) and the control wire (11) to the distal end of the control wire (11).

11. The branch sheath according to claim 1, wherein the restraining coil (24) has a length greater than a length of the first fixing coil (23), a length of the part of the restraining coil (24) extending out of the sleeving is greater than or equal to 15 mm, and a part of the first fixing coil (23) extends out of the sleeving and has a length greater than or equal to 10 mm.

12. The branch sheath according to claim 1, wherein the sleeving is a covered sleeving (22).

13. An intravascular stent delivery device, comprising a guide wire (32) for main stent and at least one branch sheath as claimed in any one of claims 1-12; wherein the guide wire (32) extends along an axial direction of the main stent (31) through the first fixing coil (23) connected to the main stent (31).

14. The intravascular stent delivery device according to claim 13, wherein a length of the first fixing coil (23) is greater than or equal to an axial length of the compressed branch stent (41) plus a diameter of the compressed main stent (31).

## Patentansprüche

1. Eine Abzweighülle, umfassend ein Verbindungsrohr (21), eine Hülle, einen Steuerdraht (11), eine Rückhaltespule (24) und eine erste Fixierspule (23); wobei
ein distales Ende von dem Verbindungsrohr (21) mit einem proximalen Ende von der Hülle verbunden ist; der Steuerdraht (11) sich durch das Verbindungsrohr (21) und die Hülle erstreckt, und der Steuerdraht (11) sich entlang einer Achse von dem Verbindungsrohr (21) und der Hülle (22) erstreckt, und ein proximales Ende und ein distales Ende aufweist, die sich jeweils aus dem Verbindungsrohr (21) und der Hülle heraus erstrecken;
ein proximales Ende von der Rückhaltespule (24) mit dem distalen Ende von dem Verbindungsrohr (21) verbunden ist, und ein distales Ende von der Rückhaltespule (24) durch die Hülle hindurchgeht und sich aus der Hülle heraus erstreckt, um so durch eine Öffnung von einem blanken Ende (411) von dem Abzweig-Stent (41) hindurchzugehen und sich aus dieser heraus zu erstrecken, um das blanke Ende (411) von dem Abzweig-Stent (41) zu ziehen und zu sichern; das distale Ende von dem Steuerdraht (11) sich durch einen Teil von der Rückhaltespule (24) erstreckt, der sich aus der Öffnung von dem blanken Ende (411) heraus erstreckt, und wobei der Steuerdraht (11) ausgebildet ist, aus der Rückhaltespule (24), der abgedeckten Hülle (22) und dem Verbindungsrohr (21) herausgezogen zu werden, um den Abzweig-Stent (41) freizugeben;
ein proximales Ende von der ersten Fixierspule (23) mit dem distalen Ende von dem Verbindungsrohr (21) verbunden ist, und ein distales Ende von der ersten Fixierspule (23) ausgebildet ist, sich mit einem Haupt-Stent (31) zu verbinden.

2. Die Abzweighülle nach Anspruch 1, wobei das proximale Ende von dem Steuerdraht (11) fest mit einem Stopper verbunden ist, um so zu verhindern, dass das proximale Ende von dem Steuerdraht (11) gleitet.

3. Die Abzweighülle nach Anspruch 2, wobei der Stopper lösbar mit dem proximalen Ende von dem Verbindungsrohr (21) verbunden ist.

4. Die Abzweighülle nach Anspruch 3, wobei die lösbare Verbindung eine Hülsenverbindung unter Verwendung von einem Wärmeschrumpfschlauch oder eine Biokleber-Adhäsion umfasst.

5. Die Abzweighülle nach Anspruch 3, wobei der Stopper einstückig mit dem Verbindungsrohr (21) ausgebildet ist, und eine Falte an einer Verbindung zwischen dem Stopper und dem Verbindungsrohr (21) ausgebildet ist, so dass bewirkt werden kann, dass die Verbindung entlang der Falte während der Freigabe von dem Abzweig-Stent (41) gebrochen wird und der Steuerdraht (11) herausgezogen werden kann.

6. Die Abzweighülle nach Anspruch 3, wobei der Stopper ein Wärmeschrumpfschlauch ist, der auf das proximale Ende von dem Verbindungsrohr (21) aufgeschoben ist, und das proximale Ende von dem Steuerdraht (11) in dem Stopper eingewickelt ist.

7. Die Abzweighülle nach Anspruch 1, ferner umfassend eine zweite Fixierspule (25); wobei ein proximales Ende von der zweiten Fixierspule (25) mit dem distalen Ende von dem Verbindungsrohr (21) verbunden ist, und ein distales Ende von der zweiten Fixierspule (25) sich durch die Hülle erstreckt, und wobei das distale Ende von der Rückhaltespule (24) sich aus der Öffnung von dem blanken Ende (411) heraus erstreckt, und sich ferner durch das distale Ende von der zweiten Fixierspule (25) erstreckt und an dem distalen Ende von der zweiten Fixierspule (25) eingehakt ist, um eine erste Verlängerung (241) zu bilden, durch die sich der Steuerdraht (11) erstreckt.

8. Die Abzweighülle nach einem von den Ansprüchen 1-7, wobei eine Anzahl von den ersten Fixierspulen (23) größer als oder gleich 2 ist, und die ersten Fixierspulen (23) in einem Intervall entlang einem Umfang von dem Verbindungsrohr (21) angeordnet sind.

9. Die Abzweighülle nach Anspruch 8, wobei mindestens eine von den ersten Fixierspulen (23) einen Knoten (231) aufweist; wobei das distale Ende von der Rückhaltespule (24) sich aus der Öffnung von dem blanken Ende (411) heraus erstreckt, sich ferner durch ein Loch erstreckt, das durch den Knoten (231) an einer Seite ausgebildet ist, die näher an dem Verbindungsrohr (21) liegt, und an dem Knoten (231) eingehakt ist, um eine zweite Verlängerung (242) zu bilden, durch die sich der Steuerdraht (11) erstreckt.

10. Die Abzweighülle nach Anspruch 1, wobei der Steuerdraht (11) eine axiale Länge aufweist, die größer als oder gleich 10 mm von einem Schnittpunkt (111) zwischen dem Teil von der Rückhaltespule (24), der sich aus der Öffnung von dem blanken Ende (411) heraus erstreckt, und dem Steuerdraht (11) zu dem distalen Ende von dem Steuerdraht (11) ist.

11. Die Abzweighülle nach Anspruch 1, wobei die Rückhaltespule (24) eine Länge aufweist, die größer als eine Länge von der ersten Fixierspule (23) ist, eine Länge von dem Teil von der Rückhaltespule (24), der sich aus der Hülle heraus erstreckt, größer als oder gleich 15 mm ist, und ein Teil von der ersten Fixierspule (23) sich aus der Hülle heraus erstreckt und eine Länge aufweist, die größer als oder gleich 10 mm ist.

12. Die Abzweighülle nach Anspruch 1, wobei die Hülle eine abgedeckte Hülle (22) ist.

13. Eine Intravaskuläre Stent-Abgabevorrichtung, umfassend einen Führungsdraht (32) für einen Haupt-Stent und mindestens eine Abzweighülle nach einem von den Ansprüchen 1-12; wobei der Führungsdraht (32) sich entlang einer axialen Richtung von dem Haupt-Stent (31) durch die erste Fixierspule (23) erstreckt, die mit dem Haupt-Stent (31) verbunden ist.

14. Die Intravaskuläre Stent-Abgabevorrichtung nach Anspruch 13, wobei eine Länge von der ersten Fixierspule (23) größer als oder gleich einer axialen Länge von dem komprimierten Abzweig-Stent (41) plus einem Durchmesser von dem komprimierten Haupt-Stent (31) ist.

## Revendications

1. Gaine de dérivation, comprenant un tube de raccordement (21), un gainage, un fil de commande (11), une bobine de retenue (24) et une première bobine de fixation (23) ; dans laquelle
une extrémité distale du tube de raccordement (21) est raccordée à une extrémité proximale du gainage ; le fil de commande (11) s'étend à travers le tube de raccordement (21) et le gainage, et le fil de commande (11) s'étend le long d'un axe du tube de raccordement (21) et du gainage (22), et présente une extrémité proximale et une extrémité distale s'étendant respectivement hors du tube de raccordement (21) et du gainage ;
une extrémité proximale de la bobine de retenue (24) est raccordée à l'extrémité distale du tube de raccordement (21), et une extrémité distale de la bobine de retenue (24) passe à travers et s'étend hors du gainage de manière à passer à travers et s'étendre hors d'une ouverture d'une extrémité nue (411) du stent de dérivation (41) pour tirer et assujettir l'extrémité nue (411) du stent de dérivation (41) ; l'extrémité distale du fil de commande (11) s'étend à travers une partie de la bobine de retenue (24) s'étendant hors de l'ouverture de l'extrémité nue (411), et dans laquelle le fil de commande (11) est configuré pour être retiré de la bobine de retenue (24), du gainage recouvert (22) et du tube de raccordement (21) pour libérer le stent de dérivation (41) ;
une extrémité proximale de la première bobine de fixation (23) est raccordée à l'extrémité distale du tube de raccordement (21), et une extrémité distale de la première bobine de fixation (23) est configurée pour se raccorder à un stent principal (31).

2. Gaine de dérivation selon la revendication 1, dans laquelle l'extrémité proximale du fil de commande (11) est raccordée de manière fixe à une butée de manière à empêcher l'extrémité proximale du fil de commande (11) de glisser.

3. Gaine de dérivation selon la revendication 2, dans laquelle la butée est raccordée de manière amovible à l'extrémité proximale du tube de raccordement (21).

4. Gaine de dérivation selon la revendication 3, dans laquelle le raccordement amovible comprend un raccordement par manchon utilisant un tube thermorétractable ou une adhésion par bio-colle.

5. Gaine de dérivation selon la revendication 3, dans laquelle la butée est formée d'un seul tenant avec le tube de raccordement (21), et un pli est formé au niveau d'un raccordement entre la butée et le tube de raccordement (21), de sorte que le raccordement peut être amené à se rompre le long du pli lors de la libération du stent de dérivation (41) et le fil de commande (11) peut être retiré.

6. Gaine de dérivation selon la revendication 3, dans laquelle la butée est un tube thermorétractable emmanché sur l'extrémité proximale du tube de raccordement (21), et l'extrémité proximale du fil de commande (11) est enveloppée dans la butée.

7. Gaine de dérivation selon la revendication 1, comprenant en outre une deuxième bobine de fixation (25) ; dans laquelle une extrémité proximale de la deuxième bobine de fixation (25) est raccordée à l'extrémité distale du tube de raccordement (21), et une extrémité distale de la deuxième bobine de fixation (25) s'étend à travers le gainage, et dans laquelle l'extrémité distale de la bobine de retenue (24) s'étend hors de l'ouverture de l'extrémité nue (411), et s'étend en outre à travers et est accrochée sur l'extrémité distale de la deuxième bobine de fixation (25) pour former un premier prolongement (241) à travers lequel s'étend le fil de commande (11).

8. Gaine de dérivation selon l'une quelconque des revendications 1 à 7, dans laquelle un nombre des premières bobines de fixation (23) est supérieur ou égal à 2, et les premières bobines de fixation (23) sont disposées à un intervalle le long d'une circonférence du tube de raccordement (21).

9. Gaine de dérivation selon la revendication 8, dans laquelle au moins l'une des premières bobines de fixation (23) présente un nœud (231) ; dans laquelle l'extrémité distale de la bobine de retenue (24) s'étend hors de l'ouverture de l'extrémité nue (411), s'étend en outre à travers un trou formé par le nœud (231) sur un côté plus proche du tube de raccordement (21), et est accrochée sur le nœud (231) pour former un deuxième prolongement (242) à travers lequel s'étend le fil de commande (11).

10. Gaine de dérivation selon la revendication 1, dans laquelle le fil de commande (11) présente une longueur axiale qui est supérieure ou égale à 10 mm depuis une intersection (111) entre la partie de la bobine de retenue (24) s'étendant hors de l'ouverture de l'extrémité nue (411) et le fil de commande (11) jusqu'à l'extrémité distale du fil de commande (11).

11. Gaine de dérivation selon la revendication 1, dans laquelle la bobine de retenue (24) présente une longueur supérieure à une longueur de la première bobine de fixation (23), une longueur de la partie de la bobine de retenue (24) s'étendant hors du gainage est supérieure ou égale à 15 mm, et une partie de la première bobine de fixation (23) s'étend hors du gainage et présente une longueur supérieure ou égale à 10 mm.

12. Gaine de dérivation selon la revendication 1, dans laquelle le gainage est un gainage recouvert (22).

13. Dispositif d'introduction de stent intravasculaire, comprenant un fil-guide (32) pour un stent principal et au moins une gaine de dérivation selon l'une quelconque des revendications 1 à 12 ; dans lequel le fil-guide (32) s'étend le long d'une direction axiale du stent principal (31) à travers la première bobine de fixation (23) raccordée au stent principal (31).

14. Dispositif d'introduction de stent intravasculaire selon la revendication 13, dans lequel une longueur de la première bobine de fixation (23) est supérieure ou égale à une longueur axiale du stent de dérivation (41) comprimé plus un diamètre du stent principal (31) comprimé.
